# EUROPEAN PATENT SPECIFICATION

(11) **EP 2 476 385 B1**
(45) Date of publication and mention of the grant of the patent: **10.05.2017**
(21) Application number: 10814865.1
(22) Date of filing: 25.03.2010
(51) Int. Cl.: A61B 17/56, A61M 5/31, A61M 5/178, A61M 5/20

(54) **REMOTE-CONTROLLED INJECTION APPARATUS FOR INJECTING BONE MATERIAL INTO VERTEBRAL BODY**
FERNGESTEUERTE INJEKTIONSVORRICHTUNG ZUR INJEKTION VON KNOCHENMATERIAL IN WIRBELKÖRPER
APPAREIL D'INJECTION TÉLÉCOMMANDÉ POUR INJECTION D'UN MATÉRIAU OSSEUX DANS UN CORPS VERTÉBRAL

(30) Priority: 09.09.2009 CN 200910018653
(43) Date of publication of application: 18.07.2012
(73) Proprietor: Shao, Weixing, Shandong 250014 (CN)
(72) Inventor: Shao, Weixing, Shandong 250014 (CN)
(74) Representative: Sun, Yiming
(86) International application number: PCT/CN2010/000371
(87) International publication number: WO 2011/029263

(56) References cited:
- WO-A1-2011/098744
- WO-A2-2004/014263
- WO-A2-2004/075954
- WO-A2-2008/045329
- AT-B- 410 056
- CN-U- 86 210 943
- CN-Y- 2 226 459
- CN-Y- 2 652 413
- CN-Y- 2 788 784
- FR-A1- 2 735 031
- RU-C1- 2 363 411
- US-A- 5 219 099

## Description

The present invention relates to a kind of medical device, especially a remote-controlled injection device for injecting bone materials into the centrum

### Description of the Related Art

Currently, vertebroplasty and replacement need to be performed for the patients who have vertebral compression fractures, and in the vertebroplasty surgey, some bone materials such as bone cement need to be injected into the centrum at the fracture location. In the existing technologies, the bone materials are injected directly into the centrum at the bone lesions by injector under the monitoring of radioexamination, for the fluid injection of bone materials, the operators should be extremely cautions and careful during injecting operation, once the bone materials have flown to the unwanted location by improper operation, it would cause an irreparable medical accident, so a radial monitoring should be needed for reducing accidents during the entire process of bone materials injection, therefore, it is necessary for the specific operators in the vertebroplasty surgery to be very close to the radioexamination equipment, which would cause injury for them, and it is more serious especially for the doctors frequently engaged in such operations. That is the deficiency of the existing technologies.

Remote controlled injection devices with an injector connected to a stepper motor are known from Russian patent RU 2363411 C1 and PCT patent application WO2004/075954 A2. The motor and injector are coaxial in the device of RU 2363411 C1, and the device of WO2004/075954 A2 includes a precession spike. The preamble of claims 1 and 3is based on the device of US patent 5,291,099, in which also a handle joint in the form of a drive shaft is connected to a dynamical joint in the form of a ball nut.

### Summary of the invention

The purpose of the present invention is to offer two technical embodiments of a remote-controlled injection device for injecting bone materials into centrum aiming at the shortcomings of existing technologies. In the embodiments, the specific injection operation will be completed by controlled machine, so operators can be far away from the radioexamination equipment for not only monitoring the injection situation, but also remotely controlling the injection process of injection device, then it will not appear misoperation and can ensure the operators to be avoided damaging by radiation.

The embodiment can be achieved through the following technical measures:

### The first technical embodiment:

A remote-controlled injection device for injecting bone materials into the centrum includes an injector with a piston and a precession handspike, a rotational handle is fixed at one end of the precession handspike, the feature of the embodiment lies in that it includes a base, an injector holder is fixed at one end of the base, and the said injector is fixed in the injector holder, the said rotational handle connects with the handle joint which connects with dynamical joint, and the dynamical joint connects with the projecting shaft of stepper motor which is coaxial with the precession handspike. The specific feature of the embodiment may also include that the said stepper motor is fixed on the motor mounting plate which is installed on the base, and can translate along the base or be locked. The said connection of rotational handle with handle joint is plugging the rotational handle into the handle cavity of handle joint. The connection of handle joint with dynamical joint can be fast disassembly. Optionally said injector holder has an injector fixed knob, which can be used to fasten the injector tightly within the injector holder with bolt.

### The second technical embodiment:

A remote-controlled injection device for injecting bone materials into the centrum includes an injector with a piston and a slip handspike, a driving handle is fixed at one end of the slip handspike, the feature of the embodiment lies in that it includes a base, an injector holder is fixed at one end of the base, and the said injector is fixed in the injector holder, the said driving handle connects with the handle joint which connects with dynamical joint, and the dynamical joint connects with the projecting shaft of linear stepper motor with a thread which is coaxial with the slip handspike, the linear stepper motor may be fastened on the base. The specific feature of the embodiment also includes that the said connection of driving handle with handle joint is plugging the driving handle into the handle cavity of handle joint. The connection of handle joint with dynamical joint can be disassembled fast. The said injector holder optionally has an injector fixed knob which can be used to fasten the injector tightly within the injector holder with bolt.

The beneficial effects of the embodiments can be recognized by the above narration, the precession handspike or slip handspike is in coaxial connection with stepper motor or linear stepper motor by handle joint and dynamical joint because the injector is fastened in the injector holder in the two embodiments. Based on this structure, the injecting action of injector can be controlled by stepper motor or linear stepper motor, and it is easy to achieve the remote-control of stepper motor or linear stepper motor, so the operators can be far away from the injection site to implement the injection in front of the monitoring display of radioexamination equipment, which ensures the accuracy of the operation and avoids the misoperation, and therefore greatly enhances the quality of surgery. The control for stepper motor or linear stepper motor can also achieve some operations such as fast moving forward, fast moving back, injection, stop and clearing, and the dose can be displayed in real time, and it can be set in advance. Thus, the present invention has substantive features and progress compared with existing technologies, it is also obvious for its beneficial effects of implementation.

### Description of the drawings:

FIG. 1 is the partly sectional structure view of the first embodiment of the invention.
FIG. 2 is the top structure view of the first embodiment of the invention.
FIG. 3 is the partly sectional structure view of the second embodiment of the invention.

Where, 1 is base, 2 is injector, 3 is piston, 4 is precession handspike, 5 is injector holder, 6 is injector fixed knob, 7 is hand cavity, 8 is rotational handle, 9 is handle joint, 10 is dynamical joint, 11 is stepper motor, 12 is motor mounting plate, 13 is slip handspike, 14 is driving handle, 15 is linear stepper motor.

### Detailed description of the invention

In order to clearly explain the technical features of the embodiments, the following will describe them with two embodiments and their drawings.

### The first technical embodiment:

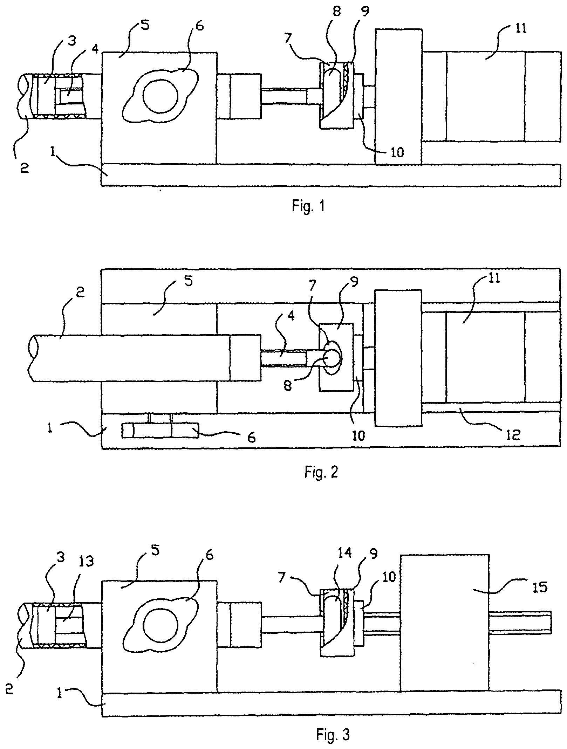

As it can be seen from FIG. 1 and FIG. 2, the remote-controlled injection device for injecting bone materials into the centrum of the invention includes a base 1, and an injector holder 5 fastened at one end of the base 1, the injector 2 with piston 3 and precession handspike 4 is tightly fastened in the injector holder 5 with injector fixed knob 6 by bolt. The rotational handle 8 at one end of precession handspike 4 connects with handle joint 9 through rotational handle 8 plugging into the handle cavity 7 of handle joint 9. There is a fast disassembled connection between handle joint 9 and dynamic joint 10, the structure of fast disassembly varies in the existing technologies, any one can be used. Dynamic joint 10 connects with the projecting shaft of the stepper motor 11, and the projecting shaft of stepper motor 11 is coaxial with the precession handspike 4. the said stepper motor 11 is fastened on the motor mounting plate 12 which is fixed on the base 1 and can translate along the base 1 or be locked.

### The second technical embodiment:

As it can be seen from FIG. 3, the remote-controlled injection equipment for injecting bone materials into centrum of the invention includes a base 1, and an injector holder 5 fastened at one end of the base 1, the injector 2 with piston 3 and precession handspike 4 is tightly fastened in the injector holder 5 with injector fixed knob 6 by bolt. The driving handle 14 at one end of slip handspike 13 connects with handle joint 9 through driving handle 14 plugging into the handle cavity 7 of handle joint 9. There is a fast disassembled connection between handle joint 9 and dynamic joint 10, the structure of fast disassembly is various in the existing technologies, any one can be used.. Dynamic joint 10 connects to the projecting shaft with thread of the linear stepper motor 15, and the projecting shaft with thread of linear stepper motor 15 is coaxial with that of the slip handspike 13, and the linear stepper motor 15 is fastened on the base 1.

## Claims

1. A remote-controlled injection device for injecting bone materials into a centrum being **characterized in that** it includes an injector (2) with a piston (3) and a precession handspike (13), a rotational handle (8) is fixed at one end of the precession handspike (4), wherein there is a base (1), and an injector holder (5) fastened at one end of the base, said injector (2) is fastened in the injector holder, said rotational handle (8) connects with a handle joint (9) which connects with a dynamical joint (10), and the dynamical joint connects with the projecting shaft of a stepper motor (11) which is coaxial with the precession handspike (4),
**characterized in that,**
wherein the connection of the rotational handle (8) with the handle joint (9) is accomplished by plugging the rotational handle into a handle cavity (7) of the handle joint (9), and the connection of the said handle joint (9) with dynamical joint (10) can be disassembled quickly.

2. The injection device of claim 1, wherein said stepper motor (11) is fixed on a motor mounting plate (12) which is fixed on the base (1) and can translate along the base (1) or be locked.

3. A remote-controlled injection device for injecting bone materials into a centrum being **characterized in that** it includes an injector (2) with a piston (3) and a slip handspike (4), a driving handle (14) is fixed at one end of the slip handspike (13), wherein there is a base (1), and an injector holder (5) fastened at one end of the base (1), the said injector (2) is fastened in the injector holder (5), the said driving handle (14) connects with a handle joint (9) which connects with a dynamical joint (10), and the dynamical joint connects to the projecting shaft, which has a thread, of a linear stepper motor (15) which is coaxial with the slip handspike (13), the linear stepper motor is fixed on the base (1),
**characterized in that,**
wherein the connection of the driving handle (14) with the handle joint (9) is accomplished by plugging the driving handle into a handle cavity (7) of the handle joint (9), and the connection of the said handle joint (9) with dynamical joint (10) can be disassembled quickly.

## Patentansprüche

1. Ferngesteuerte Injektionsvorrichtung zum Injizieren von Knochenmaterialien in ein Centrum, **dadurch gekennzeichnet, dass** die Vorrichtung einen Injektor (2) mit einem Kolben (3) und einer Präzessionshebelstange (13) enthält, ein Drehgriff (8) an einem Ende der Präzessionshebelstange (4) befestigt ist, wobei eine Basis (1) und ein Injektorhalter (5), der an einem Ende der Basis befestigt ist, vorhanden sind, wobei der Injektor (2) in dem Injektorhalter befestigt ist, der Drehgriff (8) mit einem Griffgelenk (9) verbunden ist, das mit einem dynamischen Gelenk (10) verbunden ist, und das dynamische Gelenk mit der vorspringenden Welle eines Schrittmotors (11) verbunden ist, der zu der Präzessionshebelstange (4) koaxial ist,
**dadurch gekennzeichnet, dass**
die Verbindung des Drehgriffs (8) mit dem Griffgelenk (9) durch Einstecken des Drehgriffs in einen Griffhohlraum (7) des Griffgelenks (9) erreicht wird, und sich die Verbindung des Griffgelenks (9) mit dem dynamischen Gelenk (10) rasch lösen lässt.

2. Injektionsvorrichtung nach Anspruch 1, wobei der Schrittmotor (11) an einer Motorbefestigungsplatte (12) befestigt ist, die an der Basis (1) befestigt ist und sich entlang der Basis (1) fortbewegen oder verriegelt werden kann.

3. Ferngesteuerte Injektionsvorrichtung zum Injizieren von Knochenmaterialien in ein Centrum, **dadurch gekennzeichnet, dass** die Vorrichtung einen Injektor (2) mit einem Kolben (3) und einer Gleithebelstange (4) enthält, ein Antriebsgriff (14) an einem Ende der Gleithebelstange (13) befestigt ist, wobei eine Basis (1) und ein Injektorhalter (5), der an einem Ende der Basis (1) befestigt ist, vorhanden sind, der Injektor (2) in dem Injektorhalter (5) befestigt ist, der Antriebsgriff (14) mit einem Griffgelenk (9) verbunden ist, das mit einem dynamischen Gelenk (10) verbunden ist, und das dynamische Gelenk mit der mit einem Gewinde versehenen vorspringenden Welle eines linearen Schrittmotors (15), der zu der Gleithebelstange (13) koaxial ist, verbunden ist, der lineare Schrittmotor an der Basis (1) befestigt ist,
**dadurch gekennzeichnet, dass**
die Verbindung des Antriebsgriffs (14) mit dem Griffgelenk (9) durch Einstecken des Antriebsgriffs in einen Griffhohlraum (7) des Griffgelenks (9) erreicht wird, und sich die Verbindung des Griffgelenks (9) mit dem dynamischen Gelenk (10) rasch lösen lässt.

## Revendications

1. Dispositif d'injection télécommandé pour injecter des matériaux osseux dans un centre, **caractérisé en ce qu'**il comprend un injecteur (2) doté d'un piston (3) et d'un levier de précession (13), qu'un manche rotatif (8) est fixé à une extrémité du levier de précession (4), dans lequel il y a une base (1), et un support d'injecteur (5) fixé à une extrémité de la base, ledit injecteur (2) étant fixé dans le support d'injecteur, ledit manche rotatif (8) se connectant à un raccord de manche (9) qui se connecte à un raccord dynamique (10), et que le raccord dynamique se connecte à la tige saillante d'un moteur pas à pas (11) qui est coaxial au levier de précession (4),
**caractérisé en ce que**
la connexion du manche rotatif (8) avec le raccord de manche (9) est établie en fichant le manche rotatif dans une cavité de manche (7) du raccord de manche (9), et que la connexion dudit raccord de manche (9) avec le raccord dynamique (10) peut être dissociée rapidement.

2. Dispositif d'injection selon la revendication 1, dans lequel ledit moteur pas à pas (11) est fixé sur une plaque de montage de moteur (12) qui est fixée sur la base (1) et peut glisser le long de la base (1) ou être verrouillée.

3. Dispositif d'injection télécommandé pour injection de matériaux osseux dans un centre, **caractérisé en ce qu'**il comprend un injecteur (2) avec un piston (3) et un levier glissant (4), qu'un manche de conduite (14) est fixé à une extrémité du levier glissant (13), dans lequel il y a une base (1), et un support d'injecteur (5) fixé à une extrémité de la base (1), que ledit injecteur (2) est fixé dans le support d'injecteur (5), que ledit manche de conduite (14) se connecte à un raccord de manche (9) qui se connecte à un raccord dynamique (10), et que le raccord dynamique se connecte à la tige saillante, qui comporte un filet, d'un moteur pas à pas linéaire (15) qui est coaxial au levier glissant (13), que le moteur pas à pas linéaire est fixé sur la base (1),
**caractérisé en ce que**
la connexion du manche de conduite (14) avec le raccord de manche (9) est établie en fichant le manche de conduite dans une cavité de manche (7) du raccord de manche (9), et que la connexion dudit raccord de manche (9) avec le raccord dynamique (10) peut être dissociée rapidement.
